# EUROPEAN PATENT SPECIFICATION

(11) **EP 2 059 591 B1**
(45) Date of publication and mention of the grant of the patent: **05.09.2012**
(21) Application number: 07796735.4
(22) Date of filing: 09.07.2007
(51) Int. Cl.: C11D 3/386, C12N 9/54

(54) **Dishwashing composition that contains a protease variant**
Proteasevarianten enthaltende Geschirrspülmittel
Composition pour lavage de vaisselle contenant de variantes de protéases

(30) Priority: 18.07.2006 US 831732 P
(43) Date of publication of application: 20.05.2009
(73) Proprietor: Danisco US Inc., Palo Alto, CA 94304 (US)
(72) Inventor: AUGUSTINUS, Pieter, Palo Alto, California 94304 (US); GOEDEGEBUUR, Frits, Palo Alto, California 94304 (US); POULOSE, Ayrookaran J., Palo Alto, California 94304 (US); VAN DER LAAN, Johannes Cornelis, Palo Alto, California 94304 (US)
(74) Representative: Forrest, Graham Robert
(86) International application number: PCT/US2007/015642
(87) International publication number: WO 2008/010925

(56) References cited:
- EP-A- 1 160 327
- WO-A-91/00345
- WO-A-94/02618
- US-A- 6 017 871

## Description

### FIELD OF THE INVENTION

The present invention provides dishwashing applications.

### BACKGROUND OF THE INVENTION

Typically, traditional domestic and industrial dishwashing compositions rely on a combination of high alkalinity detergent washes and chlorine bleach for cleaning and sanitizing dishware. Such systems generally perform well on bleachable stains. However, they can be deficient in removing protein-containing soils that are often present on dishware in homes, hospitals, cafeterias, catering industries, etc. In addition, very highly alkaline and chlorine-containing compositions are not considered to be consumer nor environmentally friendly.

Various attempts have been made to produce dishwashing compositions that are effective at removing proteinaceous soils. These compositions typically include proteases active under alkaline conditions (*e.g*., pH of at least 9.5). However, such compositions have significant drawbacks in that they are difficult to formulate in the liquid or gel forms commonly preferred by consumers for dishwashing detergents. In addition, alkaline dishwashing compositions are often considered to be irritants.

Some attempts have been made to produce low pH (*e.g*., pH less than 9.5) dishwashing compositions. These compositions are safer, more environmentally friendly and capable of formulation into gels and liquid forms. However, current dishwashing compositions with low pHs have proven to be very ineffective at removing proteinaceous soils, even when high concentrations of enzymes (*e.g*., proteases) are formulated within the dishwashing compositions.

Thus, there remains a need in the art for dishwashing compositions that are highly effective in removing proteinaceous soils from dishware. In addition, there remains a need for dishwashing compositions that are more environmentally and consumer friendly and are in a form that is easy to use and cost-effective.

### SUMMARY OF THE INVENTION

The present invention provides a dishwashing method, comprising the steps of providing a dishwashing composition and dishware in need of cleaning; and contacting said dishware with said dishwashing composition under conditions effective to provide cleaning of said dishware;
wherein said dishwashing composition comprises a modified subtilisin, wherein said modified subtilisin has at least 70% homology with the sequence: 
AQSVPWGISRVQAPAAHNRGLTGSGVKVAVLDTGISTHPDLNIRGGASFVPGEPSTQ

DGNGHGTHVAGTIAALNNSIGVLGVAPNAELYAVKVLGASGSGSVSSIAQGLEWAGN

NGMHVANLSLGSPSPSATLEQAVNSATSRGVLVVAASGNSGAGSISYPARYANAMAV

GATDQNNNRASFSQYGAGLDIVAPGVNVQSTYPGSTYASLNGTSMATPHVAGAAALV

KQKNPSWSNVQIRNHLKNTATSLGSTNLYGSGLVNAEAATR
 (referred to herein as SEQ ID NO: 2) and comprises either
(i) the substitution G116V and at least one additional mutation; or
(ii) the substitution S126R.

The modified subtilisin may have at least 75%, at least 80%, at least 85%, at least 90%, at least 95% or at least 99% homology with SEQ ID NO: 2. The modified subtilisin may provide improved wash performance and/or improved stability as compared to wild-type PB92 protease.

The modified subtilisin may comprise substitutions made at positions G116, S126, P127 and S128.

When the modified subtilisin comprises the substitution G116V, the additional mutation may, for example, be selected from the group consisting of S126F, S126L, S 126N, S 126R, S 126V, P127E, P127L, P127M, P127N, P127L, P127Q, P127S, S128A, S128K, S128P, S128T, S128V, and S160D.

The modified subtilisin comprises substitutions made at positions S 126, P127 and S128. In such cases, the substitutions may, for example, be selected from the group consisting of S126C, S126R, P127Q, P127R, S128D and S128G.

Modified subtilisins suitable for use in the present invention include those designated as 049, 045, 046, 047/048, 050, 053, 054, 055/056, 057, 058 and 059, having the substitutions as set forth in Table 1, herein.

For example, the amino acid sequence of said modified subtilisin may be: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSISYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR (049)
 or

AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSIDYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR (046).

### DESCRIPTION OF THE FIGURES

Figure 1A shows the construction of the mutation vector containing the PB92 protease gene.

Figure 1B provides a schematic showing the mutation procedure used in some embodiments of the present invention.

Figure 1C shows the construction of an expression vector containing a mutant PB92 protease gene.

Figure 2 provides the nucleotide sequence of the PB92 protease gene (SEQ ID NO:1) and the amino acid sequence (SEQ ID NO:2) of the encoded pre-protein, pro-protein and mature protein.

### DESCRIPTION OF THE INVENTION

The present invention provides methods and compositions comprising at least one modified subtiliser, or mutants protease for dishwashing applications. (The terms are used interchangeably)

Unless otherwise indicated, the practice of the present invention involves conventional techniques commonly used in molecular biology, microbiology, protein purification, protein engineering, protein and DNA sequencing, recombinant DNA fields, and industrial enzyme use and development, all of which are within the skill of the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, definitions for a number of terms are provided below.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Markham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

It is intended that every maximum numerical limitation given throughout this specification include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "compatible," means that the cleaning composition materials do not reduce the enzymatic activity of the protease enzyme(s) provided herein to such an extent that the protease(s) is/are not effective as desired during normal use situations. Specific cleaning composition materials are exemplified in detail hereinafter.

As used herein, "effective amount of enzyme" refers to the quantity of enzyme necessary to achieve the enzymatic activity required in the specific application. Such effective amounts are readily ascertained by one of ordinary skill in the art and are based on many factors, such as the particular enzyme variant used, the cleaning application, the specific composition of the cleaning composition, and whether a liquid or dry (*e.g*., granular) composition is required, and the like.

As used herein, "having improved properties" used in connection with "mutant proteolytic enzymes," refers to proteolytic enzymes with improved performance and/or improved stability with retained performance, relative to the corresponding wild-type protease. In some particularly preferred embodiments, the improved properties are selected from the group consisting of improved dishwash performance and improved stability, as well as the combination of improved dishwash performance and improved stability.

As used herein, the phrase "detergent stability" refers to the stability of a detergent composition. In some embodiments, the stability is assessed during the use of the detergent, while in other embodiments, the term refers to the stability of a detergent composition during storage.

The term "improved stability" is used to indicate better stability of mutant protease(s) in compositions during storage and/or better stability in the sud. In preferred embodiments, the mutant protease(s) exhibit improved stability in dish care detergents during storage and/or improved stability in the sud, which includes stability against oxidizing agents, sequestering agents, autolysis, surfactants and high alkalinity, relative to the corresponding wild-type enzyme.

As used herein, the phrase, "stability to proteolysis" refers to the ability of a protein (*e.g*., an enzyme) to withstand proteolysis. It is not intended that the term be limited to the use of any particular protease to assess the stability of a protein.

As used herein, "oxidative stability" refers to the ability of a protein to function under oxidative conditions. In particular, the term refers to the ability of a protein to function in the presence of various concentrations of H₂O₂, peracids and other oxidants. Stability under various oxidative conditions can be measured either by standard procedures known to those in the art and/or by the methods described herein. A substantial change in oxidative stability is evidenced by at least about a 5% or greater increase or decrease (in most embodiments, it is preferably an increase) in the half-life of the enzymatic activity, as compared to the enzymatic activity present in the absence of oxidative compounds.

As used herein, "pH stability" refers to the ability of a protein to function at a particular pH. In general, most enzymes have a finite pH range at which they will function. In addition to enzymes that function in mid-range pHs (i.e., around pH 7), there are enzymes that are capable of working under conditions with very high or very low pHs. Stability at various pHs can be measured either by standard procedures known to those in the art and/or by the methods described herein. A substantial change in pH stability is evidenced by at least about 5% or greater increase or decrease (in most embodiments, it is preferably an increase) in the half-life of the enzymatic activity, as compared to the enzymatic activity at the enzyme's optimum pH. However, it is not intended that the present invention be limited to any pH stability level nor pH range.

As used herein, "thermal stability" refers to the ability of a protein to function at a particular temperature. In general, most enzymes have a finite range of temperatures at which they will function. In addition to enzymes that work in mid-range temperatures (*e.g*., room temperature), there are enzymes that are capable of working in very high or very low temperatures. Thermal stability can be measured either by known procedures or by the methods described herein. A substantial change in thermal stability is evidenced by at least about 5% or greater increase or decrease (in most embodiments, it is preferably an increase) in the half-life of the catalytic activity of a mutant when exposed to given temperature. However, it is not intended that the present invention be limited to any temperature stability level nor temperature range.

As used herein, the term "chemical stability" refers to the stability of a protein (*e.g*., an enzyme) towards chemicals that may adversely affect its activity. In some embodiments, such chemicals include, but are not limited to hydrogen peroxide, peracids, anionic detergents, cationic detergents, non-ionic detergents, chelants, etc. However, it is not intended that the present invention be limited to any particular chemical stability level nor range of chemical stability.

As used herein, the terms "purified" and "isolated" refer to the removal of contaminants from a sample. For example, an enzyme of interest is purified by removal of contaminating proteins and other compounds within a solution or preparation that are not the enzyme of interest. In some embodiments, recombinant enzymes of interest are expressed in bacterial or fungal host cells and these recombinant enzymes of interest are purified by the removal of other host cell constituents; the percent of recombinant enzyme of interest polypeptides is thereby increased in the sample.

As used herein, "protein of interest," refers to a protein (*e.g*., an enzyme or "enzyme of interest") which is being analyzed, identified and/or modified. Naturally-occurring, as well as recombinant (*e.g*., mutant) proteins find use in the present invention.

As used herein, "protein" refers to any composition comprised of amino acids and recognized as a protein by those of skill in the art. The terms "protein," "peptide" and polypeptide are used interchangeably herein. Wherein a peptide is a portion of a protein, those skilled in the art understand the use of the term in context.

As used herein, functionally and/or structurally similar proteins are considered to be "related proteins." In some embodiments, these proteins are derived from a different genus and/or species, including differences between classes of organisms (*e.g*., a bacterial protein and a fungal protein). In some embodiments, these proteins are derived from a different genus and/or species, including differences between classes of organisms (*e.g*., a bacterial enzyme and a fungal enzyme). In additional embodiments, related proteins are provided from the same species. Indeed, it is not intended that the present invention be limited to related proteins from any particular source(s). In addition, the term "related proteins" encompasses tertiary structural homologs and primary sequence homologs (*e.g*., the enzymes of the present invention). In further embodiments, the term encompasses proteins that are immunologically cross-reactive.

As used herein, the term "derivative" refers to a protein which is derived from a protein by addition (*i.e*., insertion) of one or more amino acids to either or both the C- and N-terminal end(s), substitution of one or more amino acids at one or a number of different sites in the amino acid sequence, and/or deletion of one or more amino acids at either or both ends of the protein or at one or more sites in the amino acid sequence, and/or insertion of one or more amino acids at one or more sites in the amino acid sequence. The preparation of a protein derivative is preferably achieved by modifying a DNA sequence which encodes for the native protein, transformation of that DNA sequence into a suitable host, and expression of the modified DNA sequence to form the derivative protein.

Related (and derivative) proteins comprise "variant proteins." In some preferred embodiments, variant proteins differ from a parent protein and one another by a small number of amino acid residues. The number of differing amino acid residues may be one or more, preferably 1, 2, 3, 4, 5, 10, 15, 20, 30, 40, 50, or more amino acid residues. In some preferred embodiments, the number of different amino acids between variants is between 1 and 10. In some particularly preferred embodiments, related proteins and particularly variant proteins comprise at least about 35%, 40%, 45%, 50%, 55%, 60%, 65%, 70%, 75%, 80%, 85%, 90%, 95%, 97%, 98%, or 99% amino acid sequence identity. Additionally, a related protein or a variant protein as used herein, refers to a protein that differs from another related protein or a parent protein in the number of prominent regions. For example, in some embodiments, variant proteins have 1, 2, 3, 4, 5, or 10 corresponding prominent regions that differ from the parent protein.

Several methods are known in the art that are suitable for generating variants of the protease enzymes of the present invention, including but not limited to site-saturation mutagenesis, scanning mutagenesis, insertional mutagenesis, random mutagenesis, site-directed mutagenesis, and directed-evolution, as well as various other recombinatorial approaches.

As used herein, "expression vector" refers to a DNA construct containing a DNA sequence that is operably linked to a suitable control sequence capable of effecting the expression of the DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid," "expression plasmid," and "vector" are often used interchangeably, as the plasmid is the most commonly used form of vector at present. However, the invention is intended to include such other forms of expression vectors that serve equivalent functions and which are, or become, known in the art.

In some preferred embodiments, the protease gene is ligated into an appropriate expression plasmid. The cloned protease gene is then used to transform or transfect a host cell in order to express the protease gene. This plasmid may replicate in hosts in the sense that it contains the well-known elements necessary for plasmid replication or the plasmid may be designed to integrate into the host chromosome. The necessary elements are provided for efficient gene expression (*e.g*., a promoter operably linked to the gene of interest). In some embodiments, these necessary elements are supplied as the gene's own homologous promoter if it is recognized, (*i.e*., transcribed by the host), and a transcription terminator that is exogenous or is supplied by the endogenous terminator region of the protease gene. In some embodiments, a selection gene such as an antibiotic resistance gene that enables continuous cultural maintenance of plasmid-infected host cells by growth in antimicrobial-containing media is also included.

The following cassette mutagenesis method may be used to facilitate the construction of the protease variants of the present invention, although other methods may be used.

First, as described herein, a naturally-occurring gene encoding the protease is obtained and sequenced in whole or in part. Then, the sequence is scanned for a point at which it is desired to make a mutation (deletion, insertion or substitution) of one or more amino acids in the encoded protease. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the protease gene may be used, provided the gene fragments generated by restriction digestion can be reassembled in proper sequence. If restriction sites are not present at locations within a convenient distance from the selected point (from 10 to 15 nucleotides), such sites are generated by substituting nucleotides in the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA and/or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

As used herein, "corresponding to," refers to a residue at the enumerated position in a protein or peptide, or a residue that is analogous, homologous, or equivalent to an enumerated residue in a protein or peptide.

As used herein, "corresponding region," generally refers to an analogous position along related proteins or a parent protein.

The terms "nucleic acid molecule encoding," "nucleic acid sequence encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the term "analogous sequence" refers to a sequence within a protein that provides similar function, tertiary structure, and/or conserved residues as the protein of interest (i.e., typically the original protein of interest). For example, in epitope regions that contain an alpha helix or a beta sheet structure, the replacement amino acids in the analogous sequence preferably maintain the same specific structure. The term also refers to nucleotide sequences, as well as amino acid sequences. In some embodiments, analogous sequences are developed such that the replacement amino acids result in a variant enzyme showing a similar or improved function. In some preferred embodiments, the tertiary structure and/or conserved residues of the amino acids in the protein of interest are located at or near the segment or fragment of interest. Thus, where the segment or fragment of interest contains, for example, an alpha-helix or a betasheet structure, the replacement amino acids preferably maintain that specific structure.

As used herein, "homologous protein" refers to a protein (*e.g*., protease) that has similar action and/or structure, as a protein of interest (*e.g*., a protease from another source). It is not intended that homologs be necessarily related evolutionarily. Thus, it is intended that the term encompass the same or similar enzyme(s) (*i.e*., in terms of structure and function) obtained from different species. In some preferred embodiments, it is desirable to identify a homolog that has a quaternary, tertiary and/or primary structure similar to the protein of interest, as replacement for the segment or fragment in the protein of interest with an analogous segment from the homolog will reduce the disruptiveness of the change. In some embodiments, homologous proteins have induced similar immunological response(s) as a protein of interest.

As used herein, "homologous genes" refers to at least a pair of genes from different species, which genes correspond to each other and which are identical or very similar to each other. The term encompasses genes that are separated by speciation (*i.e*., the development of new species) (*e.g*., orthologous genes), as well as genes that have been separated by genetic duplication (*e.g*., paralogous genes). These genes encode "homologous proteins."

As used herein, "ortholog" and "orthologous genes" refer to genes in different species that have evolved from a common ancestral gene (*i.e*., a homologous gene) by speciation. Typically, orthologs retain the same function during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "wild-type" and "native" proteins are those found in nature. The terms "wild-type sequence," and "wild-type gene" are used interchangeably herein, to refer to a sequence that is native or naturally occurring in a host cell. In some embodiments, the wild-type sequence refers to a sequence of interest that is the starting point of a protein engineering project. The genes encoding the naturally-occurring protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

The term "recombinant DNA molecule" as used herein refers to a DNA molecule that is comprised of segments of DNA joined together by means of molecular biological techniques.

The term "recombinant oligonucleotide" refers to an oligonucleotide created using molecular biological manipulations, including but not limited to, the ligation of two or more oligonucleotide sequences generated by restriction enzyme digestion of a polynucleotide sequence, the synthesis of oligonucleotides (*e.g*., the synthesis of primers or oligonucleotides) and the like.

The degree of homology between sequences may be determined using any suitable method known in the art (*See e.g*., Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

For example, PILEUP is a useful program to determine sequence homology levels. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle, (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps. Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). One particularly useful BLAST program is the WU-BLAST-2 program (See, Altschul et al., Meth. Enzymol.., 266:460-480 [1996]). parameters "W," "T," and "X" determine the sensitivity and speed of the alignment. The BLAST program uses as defaults a wordlength (W) of 11, the BLOSUM62 scoring matrix *(See,* Henikoffand Henikoff, Proc. Natl. Acad. Sci. USA 89:10915 [1989]) alignments (B) of 50, expectation (E) of 10, M'5, N'-4, and a comparison of both strands.

As used herein, "percent (%) nucleic acid sequence identity" is defined as the percentage of nucleotide residues in a candidate sequence that is identical with the nucleotide residues of the sequence.

As used herein, the term "hybridization" refers to the process by which a strand of nucleic acid joins with a complementary strand through base pairing, as known in the art.

As used herein, the phrase "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm-5° C (5° below the Tm of the probe); "high stringency" at about 5-10° below the Tm; "intermediate stringency" at about 10-20° below the Tm of the probe; and "low stringency" at about 20-25° below the Tm. Alternatively, or in addition, hybridization conditions can be based upon the salt or ionic strength conditions of hybridization and/or one or more stringency washes. For example, 6xSSC = very low stringency; 3xSSC = low to medium stringency; 1xSSC = medium stringency; and 0.5xSSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe.

For applications requiring high selectivity, it is typically desirable to use relatively stringent conditions to form the hybrids (e.g., relatively low salt and/or high temperature conditions are used).

The phrases "substantially similar and "substantially identical" in the context of at least two nucleic acids or polypeptides typically means that a polynucleotide or polypeptide comprises a sequence that has at least about 50% identity, more preferably at least about 60% identity, still more preferably at least about 75% identity, more preferably at least about 80% identity, yet more preferably at least about 90%, still more preferably about 95%, most preferably about 97% identity, sometimes as much as about 98% and about 99% sequence identity, compared to the reference (i.e., wild-type) sequence. Sequence identity may be determined using known programs such as BLAST, ALIGN, and CLUSTAL using standard parameters. *(See e.g.,* Altschul, et al., J. Mol. Biol. 215:403-410 [1990]; Henikoff et al., Proc. Natl. Acad. Sci. USA 89:10915 [1989]; Karin et al., Proc. Natl. Acad. Sci USA 90:5873 [1993]; and Higgins et al., Gene 73:237 - 244 [1988]). Software for performing BLAST analyses is publicly available through the National Center for Biotechnology Information. Also, databases may be searched using FASTA (Pearson et al., Proc. Natl. Acad. Sci. USA 85:2444-2448 [1988]). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (e.g., within a range of medium to high stringency).

As used herein, "equivalent residues" refers to proteins that share particular amino acid residues. For example, equivalent resides may be identified by determining homology at the level of tertiary structure for a protein (e.g., protease) whose tertiary structure has been determined by x-ray crystallography. Equivalent residues are defined as those for which the atomic coordinates of two or more of the main chain atoms of a particular amino acid residue of the protein having putative equivalent residues and the protein of interest are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/analysis.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "host cells" are generally prokaryotic or eukaryotic hosts which are transformed or transfected with vectors constructed using recombinant DNA techniques known in the art. Transformed host cells are capable of either replicating vectors encoding the protein variants or expressing the desired protein variant. In the case of vectors which encode the preor prepro-form of the protein variant, such variants, when expressed, are typically secreted from the host cell into the host cell medium.

The term "introduced" in the context of inserting a nucleic acid sequence into a cell, means transformation, transduction or transfection. Means of transformation include, but are not limited, to any suitable methods known in the art, such as protoplast transformation, calcium chloride precipitation, electroporation, naked DNA and the like, as known in the art. *(See,* Chang and Cohen, Mol. Gen. Genet., 168:111 - 115 [1979]; Smith et al., Appl. Env. Microbiol., 51:634 [1986]; and the review article by Ferrari et al., in Harwood, Bacillus, Plenum Publishing Corporation, pp. 57-72 [1989]).

The term "promoter/enhancer" denotes a segment of DNA which contains sequences capable of providing both promoter and enhancer functions. The enhancer/promoter may be "endogenous" or "exogenous" or "heterologous." An endogenous enhancer/promoter is one which is naturally linked with a given gene in the genome. An exogenous (heterologous) enhancer/promoter is one which is placed in juxtaposition to a gene by means of genetic manipulation (*i.e*., molecular biological techniques).

The presence of "splicing signals" on an expression vector often results in higher levels of expression of the recombinant transcript. Splicing signals mediate the removal of introns from the primary RNA transcript and consist of a splice donor and acceptor site (Sambrook et al., Molecular Cloning: A Laboratory Manual, 2nd ed., Cold Spring Harbor Laboratory Press, New York [1989], pp. 16.7-16.8).

The term "stable transfection" or "stably transfected" refers to the introduction and integration of foreign DNA into the genome of the transfected cell. The term "stable transfectant" refers to a cell which has stably integrated foreign or exogenous DNA into the genomic DNA of the transfected cell.

The terms "selectable marker" or "selectable gene product" as used herein refer to the use of a gene which encodes an enzymatic activity that confers resistance to an antibiotic or drug upon the cell in which the selectable marker is expressed.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g*., an inhibitor of an inhibitable enzyme) results in the amplification of either the endogenous gene encoding the gene product required for growth in the presence of the drug or by amplification of exogenous (*i.e*., input) sequences encoding this gene product, or both. Selection of cells by growth in the presence of a drug (*e.g*., an inhibitor of an inhibitable enzyme) may result in the amplification of either the endogenous gene encoding the gene product required for growth in the presence of the drug or by amplification of exogenous (*i.e*., input) sequences encoding this gene product, or both.

"Amplification" is a special case of nucleic acid replication involving template specificity. It is to be contrasted with non-specific template replication (*i.e*., replication that is template-dependent but not dependent on a specific template). Template specificity is here distinguished from fidelity of replication (*i.e*., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "co-amplification" refers to the introduction into a single cell of an amplifiable marker in conjunction with other gene sequences (*i.e*., comprising one or more non-selectable genes such as those contained within an expression vector) and the application of appropriate selective pressure such that the cell amplifies both the amplifiable marker and the other, non-selectable gene sequences. The amplifiable marker may be physically linked to the other gene sequences or alternatively two separate pieces of DNA, one containing the amplifiable marker and the other containing the non-selectable marker, may be introduced into the same cell.

As used herein, the terms "amplifiable marker," "amplifiable gene," and "amplification vector" refer to a marker, gene or a vector encoding a gene which permits the amplification of that gene under appropriate growth conditions.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g.,* Kacian et al., Proc. Natl. Acad. Sci. USA 69:3038 [1972]). Other nucleic acids are not replicated by this amplification enzyme. Similarly, in the case of T7 RNA polymerase, this amplification enzyme has a stringent specificity for its own promoters (*See*, Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See*, Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (*i.e*., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers depend on many factors, including temperature, source of primer and the use of the method.

As used herein, the term "probe" refers to an oligonucleotide (*i.e*., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (*e.g*., ELISA, as well as enzyme-based histochemical assays), fluorescent, radioactive, and luminescent systems. It is not intended that the present invention be limited to any particular detection system or label.

As used herein, the term "target," when used in reference to amplification methods (*e.g*., the polymerase chain reaction), refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195, 4,683,202, and 4,965,188, which include methods for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (*i.e*., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplifed".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR, it is possible to amplify a single copy of a specific target sequence in genomic DNA to a level detectable by several different methodologies (*e.g*., hybridization with a labeled probe; incorporation of biotinylated primers followed by avidin-enzyme conjugate detection; incorporation of 32P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the terms "restriction endonucleases" and "restriction enzymes" refer to bacterial enzymes, each of which cut double-stranded DNA at or near a specific nucleotide sequence.

As used herein, "surface property" is used in reference to an electrostatic charge, as well as properties such as the hydrophobicity and/or hydrophilicity exhibited by the surface of a protein.

As used herein, the terms "detergent composition" and "detergent formulation" are used in reference to mixtures which are intended for use in a wash medium for the cleaning of soiled objects. In preferred embodiments, the term is used in reference to detergents used to clean dishes, cutlery, etc. (*e.g*., "dishwashing detergents"). It is not intended that the present invention be limited to any particular detergent formulation or composition. Indeed, it is intended that in addition to detergents that contain at least one protease of the present invention, the term encompasses detergents that contain surfactants, transferase(s), hydrolytic enzymes, oxido reductases, builders, bleaching agents, bleach activators, bluing agents and fluorescent dyes, caking inhibitors, masking agents, enzyme activators, antioxidants, and solubilizers.

As used herein, "dishwashing composition" refers to all forms of compositions for cleaning dishware, including cutlery, including but not limited to granular and liquid forms. It is not intended that the present invention be limited to any particular type or dishware composition. Indeed, the present invention finds use in cleaning dishware (*e.g*., dishes, including, but not limited to plates, cups, glasses, bowls, etc.) and cutlery (*e.g*., utensils, including but not limited to spoons, knives, forks, serving utensils, etc.) of any material, including but not limited to ceramics, plastics, metals, china, glass, acrylics, etc. The term "dishware" is used herein in reference to both dishes and cutlery.

As used herein, "wash performance" of mutant protease refers to the contribution of a mutant protease enzyme to dishwashing that provides additional cleaning performance to the detergent without the addition of the mutant protease to the composition. Wash performance is compared under relevant washing conditions.

The term "relevant washing conditions" is used herein to indicate the conditions, particularly washing temperature, time, washing mechanics, sud concentration, type of detergent and water hardness, actually used in households in a dish detergent market segment.

The term "improved wash performance" is used to indicate that a better end result is obtained in stain removal from dishware and/or cutlery under relevant washing conditions, or that less mutant protease, on weight basis, is needed to obtain the same end result relative to the corresponding wild-type enzyme.

The term "retained wash performance" is used to indicate that the wash performance of a mutant protease enzyme, on weight basis, is at least 80% relative to the corresponding wild-type protease under relevant washing conditions.

Wash performance of proteases is conveniently measured by their ability to remove certain representative stains under appropriate test conditions. In these test systems, other relevant factors, such as detergent composition, sud concentration, water hardness, washing mechanics, time, pH, and/or temperature, can be controlled in such a way that conditions typical for household application in a certain market segment are imitated. The laboratory application test system described herein is representative for household application when used on proteolytic enzymes modified through DNA mutagenesis. Thus, the methods provided herein facilitate the testing of large amounts of different enzymes and the selection of those enzymes which are particularly suitable for a specific type of detergent application. In this way "tailor made" enzymes for specific application conditions are easily selected.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed. -

Some bacterial serine proteases are referred to as "subtilisins." Subtilisins comprise the serine proteases of *Bacillus subtilis, Bacillus amyloliquefaciens* ("subtilisin BPN"'), and *Bacillus licheniformis* ("subtilisin Carlsberg") (*See e.g.,* Markland and Smith, in Boyer (ed.), Enzymes. The (Boyer, ed.) vol. 3, pp.561-608, Academic Press, New York, [1971]). *Bacillus* strains such as alkalophilic *Bacillus* strains produce other proteases. Examples of the latter category include such serine proteases as MAXACAL® protease (also referred to herein as "PB92 protease", isolated from *Bacillus* nov. spec. PB92), and SAVINASE® protease. Additional proteases, include but are not limited to PROPERASE® protease.

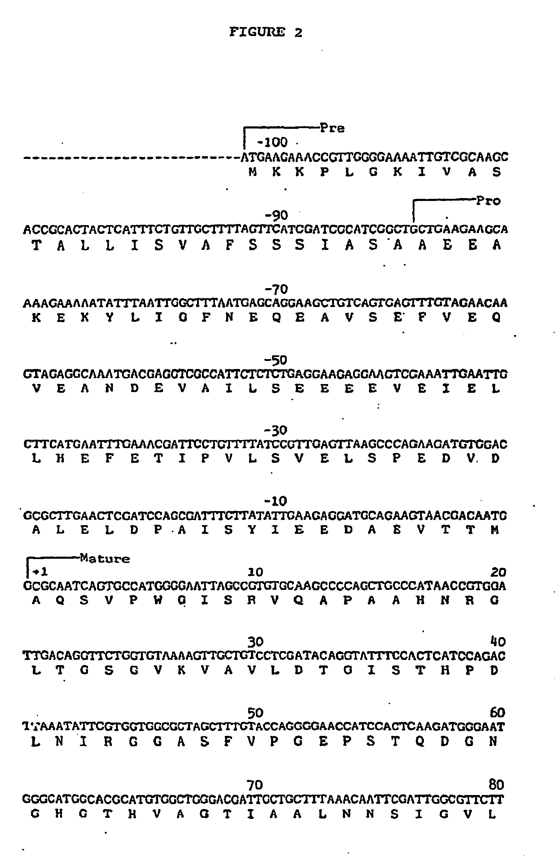

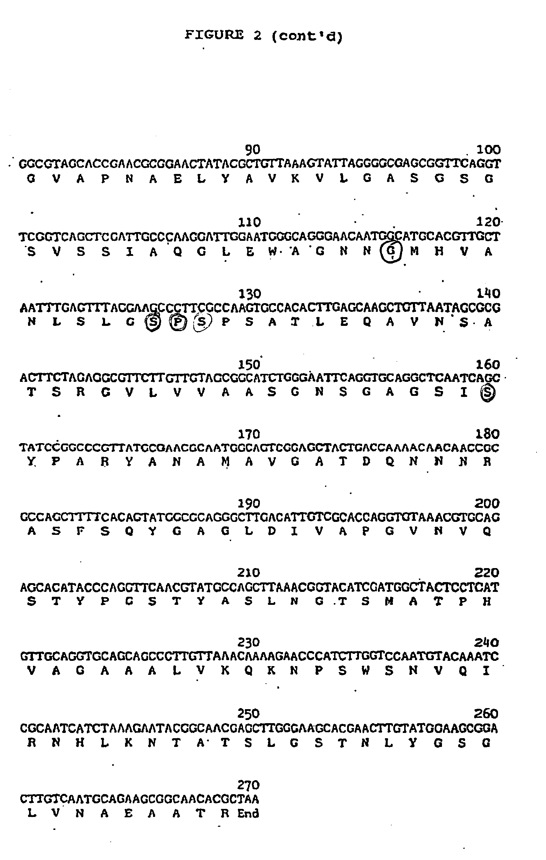

The amino acid (SEQ ID NO:2) and DNA sequences (SEQ ID NO:1) of the PB92 protease are shown in Figure 2. The mature protease consists of 269 amino acids, with a molecular weight of about 27,000 Daltons, and an isoelectric point in the high alkaline range. The activity of PB92 protease on protein substrate is expressed in Alkaline Delft Units (ADU). The activity in ADU is determined according to the method described in British Patent Specification No. 1,353,317 except that the pH was changed from 8.5 to 10.0. Purified PB92 protease has an activity of 21,000 ADU per mg. The turnover number (^{k}cat) measured on casein is 90 sec⁻¹mol⁻¹.

The specific activity of purified preparations of subtilisin Carlsberg *(See,* Delange and Smith, J. Biol. Chem., 243:2184 [1968]), amounts to 10,000 ADU/mg and of subtilisin BPN' (Matsubara et al., J. Biol. Chem., 240:1125 [1965]) to 7,000 ADU/mg. Besides the above-mentioned parameters such as specific activity and turnover number (kcat). PB92 protease distinguishes itself from proteases like Carlsberg subtilisin, subtilisin BPN' and other proteases formulated in detergents (*e.g*. MAXATASE® and ALCALASE®) in having a high positive charge, which can be visualized by gel electrophoresis of the native protein.

Since the PB92 protease is active in stain removing at alkaline pH values, it is commonly used as a detergent additive, together with detergent ingredients such as surfactants, builders and oxidizing agents. The latter agents are mostly used in powder form. PB92 protease has a high stain removing efficiency as compared to other proteases, such as the aforementioned subtilisins. This means that less PB92 protease is needed to achieve the same wash performance. Sensitivity to oxidation is an important drawback of the PB92 protease and all other known serine proteases used for application in detergents (*See e.g.,* Stauffer et al., J. Biol. Chem., 244:5333-5338 [1969]; and Estell et al., J. Biol. Chem., 263:6518-6521 [1985]). Oxidation of PB92 protease by either H₂O₂ or peracids generated by the activator system, containing perborate-tetrahydrate and TAED, creates an enzyme with a specific activity of 50% and 10%, respectively, on ADU/mg, compared to non-oxidized PB92 protease.

Suitable host strains for production of mutant proteases include transformable microorganisms in which expression of the protease can be achieved. Specifically host strains of the same species or genus from which the protease is derived, are suitable, such as a *Bacillus* strain, preferably an alkalophilic *Bacillus* strain and most preferably *Bacillus* nov. spec. PB92 or a mutant thereof, having substantially the same properties. Also, *B. subtilis, B. licheniformis* and *B. amyloliquefaciens* strains are among the preferred strains. Other suitable and preferred host strains include those strains which are substantially incapable of producing extracellular proteolytic enzymes prior to the transformation with a mutant gene. Of particular interest are protease deficient *Bacillus* host strains, such as a protease deficient derivative of *Bacillus* nov. spec. PB92. Expression of the proteases is obtained by using expression signals that function in the selected host organism. Expression signals include sequences of DNA regulating transcription and translation of the protease genes. Proper vectors are able to replicate at sufficiently high copy numbers in the host strain of choice or enable stable maintenance of the protease gene in the host strain by chromosomal integration.

The mutant proteolytic enzymes used in the invention are prepared by cultivating, under appropriate fermentation conditions, a transformed host strain comprising the desired mutant proteolytic gene or genes, and recovering the produced enzymes.

Preferably, the proteases being expressed are secreted into the culture medium, which facilitates their recovery, or in the case of gram negative bacterial host strains into the periplasmic space. For secretion a suitable amino terminal signal sequence is employed, preferably the signal sequence encoded by the original gene if this is functional in the host strain of choice.

The properties of the naturally occurring or naturally mutated detergent proteases are enhanced by introducing a variety of mutations in the enzyme. For the most part, the mutations are substitutions, either conservative or non-conservative, although deletions and insertions also find use in some embodiments.

For conservative substitutions the following table find use:
Aliphatic
   Neutral
      Non-polar (G, A, P, L, I, V)
      Polar (C, M, S, T, N, Q)
   Charged
      Anionic (D, E)
      Cationic (K, R)
Aromatic
   (F, H, W, Y)
where any amino acid may be substituted with any other amino acid in the same category, particularly on the same line. In addition, the polar amino acids N, Q may substitute or be substituted for by the charged amino acids. For the purposes of the present invention, substitutions resulting in increased anionic character of the protease, particularly at sites not directly involved with the active site are of particular interest.

Regions of particular interest for mutation are those amino acids within 4 A distance from the inhibitor molecule Eglin C, when Eglin C is bound to the active site.

The following numbering is based on PB92 protease, but the considerations are relevant to other serine proteases having a substantially homologous structure, particularly those having greater than about 70% homology, more particularly, having greater than about 90% homology. Positions of particular interest include 32, 33, 48-54, 58-62, 94-107, 116, 123-133, 150, 152-156, 158-161, 164, 169, 175-186, 197, 198, 203-216 (PB92 numbering), as most of these positions are available for direct interaction with a proteinaceous substrate. Usually, the amino acids at positions 32, 62, 153 and 215 are not substituted, since mutations at these sites tend to degrade wash performance. In some most particularly preferred embodiments, mutations are made at positions 116, 126, 127, and 128 (PB92 numbering). In alternative embodiments, an additional mutation is made at position 160.

In further embodiments, positions for substitution of particular interest include 60, 94, 97-102, 105, 116, 123-128, 150, 152, 160, 183, 203, 211, 212, 213, 214 and 216 (PB92 numbering). At some positions, the substitution changes an unstable amino acid (e.g. methionine) to an oxidatively more stable amino acid (e.g. threonine), while maintaining the general conformation and volume of the amino acid at that site. In some other embodiments, replacing the natural amino acid with almost any other amino acid, improved results are obtained, particularly in substitutions in which the hydroxylated amino acids S and/or T, are replaced with a polar or non-polar amino acid, or even an aromatic amino acid.

In some most particularly preferred embodiments, substitutions include (PB92 numbering):
G116 I, V, L
S126 any amino acid P127 any amino acid S128 any amino acid
S 160 anionic or neutral aliphatic or R
A166 charged, particularly anionic
M169 neutral aliphatic, preferably non-polar
N212 anionic
M216 aliphatic polar, particularly S, T, N, Q

Surprisingly, while many of the mutations resulted in lower specific activity of the protease with common substrates, wash performance was comparable to or enhanced in relation to the natural enzyme and in many cases storage stability was improved. In addition, the wash performance of some of the PB92 mutant proteases as compared to the native PB92 protease was found to be from about 120 to about 180 percent. Thus, the present invention provides variant proteases with much improved performance, as compared to the native protease.

In some embodiments, several mutations are combined, in order to increase the stability of a protease in detergent compositions. Several mutations that positively influence the wash of the same protease can be combined into a single mutant protease gene enabling production of possibly even further improved proteases (e.g., S126M, P127A, S128G, S 160D and G116V, S126N, P127S, S128A, S160D; PB92 numbering). Additional protease mutants are provided by combining the good wash performance properties of, for example, G116V and S160D with the stability properties of other mutations (PB92 numbering).

Useful mutants are also provided by combining any of the mutations or sets of mutations described herein. In additional embodiments, useful mutations specifically provided herein are combined with mutations at other sites. In some embodiments, these combinations result in substantial changes in the properties of the enzymes, while in other embodiments, the changes are less substantial.

The invention comprises also the use of one or more mutant proteolytic enzymes, as defined herein, in detergent composition(s) and/or in washing process(es) Finally, it will be clear that by deletions or insertions of the amino acids in the protease polypeptide chain, either created artificially by mutagenesis or naturally occurring in proteases homologous to PB92 protease, the numbering of the amino acids may change. However, it is to be understood that positions homologous to amino acid positions of PB92 protease will fall under the scope of the claims.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (revolutions per minute); H₂O (water); HCl (hydrochloric acid); aa (amino acid); bp (base pair); kb (kilobase pair); kD (kilodaltons); gm (grams); µg and ug (micrograms); mg (milligrams); ng (nanograms); µl and ul (microliters); ml (milliliters); mm (millimeters); mm (nanometers); µm and µm (micrometer); M (molar); mM (millimolar); µM and uM (micromolar); U (units); V (volts); MW (molecular weight); sec (seconds); min(s) (minute/minutes); hr(s) (hour/hours); MgCl₂ (magnesium chloride); NaCl (sodium chloride); OD₂₈₀ (optical density at 280 nm); OD₆₀₀ (optical density at 600 nm); PAGE (polyacrylamide gel electrophoresis); EtOH (ethanol); PBS (phosphate buffered saline (150 mM NaCl, 10 mM sodium phosphate buffer, pH 7.2]); SDS (sodium dodecyl sulfate); Tris (tris(hydroxymethyl)aminomethane); TAED (N,N,N'N'-tetraacetylethylenediamine); w/v (weight to volume); v/v (volume to volume); MS (mass spectroscopy); TIGR (The Institute for Genomic Research, Rockville, MD); AATCC (American Association of Textile and Coloring Chemists); SR (soil or stain removal); WFK (wfk Testgewebe GmbH, Bruggen-Bracht, Germany); Amersham (Amersham Life Science, Inc. Arlington Heights, IL); ICN (ICN Pharmaceuticals, Inc., Costa Mesa, CA); Pierce (Pierce Biotechnology, Rockford, IL); Amicon (Amicon, Inc., Beverly, MA); ATCC (American Type Culture Collection, Manassas, VA); Amersham (Amersham Biosciences, Inc., Piscataway, NJ); Becton Dickinson (Becton Dickinson Labware, Lincoln Park, NJ); BioRad (BioRad, Richmond, CA); Clontech (CLONTECH Laboratories, Palo Alto, CA); Difco (Difco Laboratories, Detroit, MI); GIBCO BRL or Gibco BRL (Life Technologies, Inc., Gaithersburg, MD); Novagen (Novagen, Inc., Madison, WI); Qiagen (Qiagen, Inc., Valencia, CA); Invitrogen (Invitrogen Corp., Carlsbad, CA); Finnzymes (Finnzymes Oy, Espoo, Finland); Macherey-Nagel (Macherey-Nagel, Easton, PA); Merieux (Institut Merieux, Codex, FR); Kelco (CP Kelco, Atlanta, GA); Genaissance (Genaissance Pharmaceuticals, Inc., New Haven, CT); DNA 2.0 (DNA 2.0, Menlo Park, CA); MIDI (MIDI Labs, Newark, DE) InvivoGen (InvivoGen, San Diego, CA); Sigma (Sigma Chemical Co., St. Louis, MO); Sorvall (Sorvall Instruments, a subsidiary of DuPont Co., Biotechnology Systems, Wilmington, DE); Stratagene (Stratagene Cloning Systems, La Jolla, CA); Roche (Hoffmann La Roche, Inc., Nutley, NJ); Agilent (Agilent Technologies, Palo Alto, CA); Minolta (Konica Minolta, Ramsey, NJ); Zeiss (Carl Zeiss, Inc., Thomwood, NY); Henkel (Henkel, GmbH, Dusseldorf, Germany); Cognis (Cognis Corp, USA, Cincinnati, OH); Finnzymes (Finnzymes Oy, Espoo, Finland); Reckitt Benckiser, Berks, United Kingdom); BASF (BASF Corp., Florham Park, NJ); IKW (Industrieverband Körperflege und Waschmittel, Frankfurt, Germany); and WFK (Testgewebe GmbH, Bruggen-Bracht, Germany).

The synthetic water with 3.00 mmol Ca+Mg (16.8 °d) used in some dishwashing experiments was prepared as follows. First, three stock solutions were prepared. Solution 1 was 800 mmol/l NaHCO₃ (67.2 g/l); solution 2 was 154.2 mmol /l MgSO₄ * 7 H₂O (38.0 g/l); and solution 3 was 446.1 mmol /l CaCl₂ * 2 H₂O (65.6 g/l). After the solutions were prepared, 50 ml each of the stock solutions 1,2 and 3 were placed in a vessel with 7 L demineralized water and then vessel then filled with additional demineralized water up to 10 L. Before use, the pH value of the synthetic water was adjusted to 7.5 with HCl or NaOH.

The following Table (Table 1) provides the mutants produced and tested during the development of the present invention. In this Table, BPN' and PB92 numbering are both provided for convenience.

| **Table 1. PB92 Mutants** | | |
|---|---|---|
| **Strain Designation** | **BPN' Numbering** | **PB92 Numbering** |
| 049* | G118V, S128L, P129Q, S130A | G116V, S126L, P127Q, S128A |
| 045* | G118V, S128N, P129S, S130A, S166D | G116V, S126N, P127S, S128A, S164D |
| 046* | G118V, S128L, P129Q, S130A, S166D | G116V, S126L, P127Q, S128A, S160D |
| 047/048* | G118V, S128V, P129E, S130K | G116V, S126V, P127E, S128K |
| 050* | G118V, S128V, P129M, S166D | G116V, S126V, P127M, S160D |
| 051/052* | S130T | S128T |
| 053 | G118V, S128F, P129L, S130T | G116V, S126F, P127L, S128T |
| 054 | G118V, S128L, P129N, S130V | G116V, S126L, P127N, S128V |
| 055/056 | G118V, S128F, P129Q | G116V, S126F, P127Q |
| 057 | G118V, S128V, P129E, S130K, S166D | G116V, S126V, P127E, S128K, S160D |
| 058 | G118V, S128R, P129S, S130P | G116V, S126R, P127S, S128P |
| 059 | S128R, P129Q, S130D | S126R, P127Q, S128D |
| 060 | S128C, P129R, S130G | S126C, P127R, S128D |

In Table 1, all strains were produced and characterized using the methods described in Examples. Strains indicated with an asterisk (*) were prepared as described in EP 0 571 049 B 1 (See, Example 1A-C). All other variants were prepared as described in Example 1D.

[0122] In Table 1, all strains were produced and characterized using the methods described in Examples. Strains indicated with an asterisk (*) were prepared as described in EP 0 571 049 B1 *(See,* Example 1A-C). All other variants were prepared as described in Example 1D.

### EXAMPLE 1

### Construction of PB92 Protease Mutants

In this Example, methods used to construct some of the PB92 mutants provided herein are described. The basic construct from which the mutagenesis work started, is referred to as "pM58," which is described in EP 0283075 and in EP 571049. The strategy followed comprised three phases:
A. Construction of Mutagenesis vector 13M1
B. Mutation Procedure
C. Construction of pM58Eco and Subcloning of the Mutated DNA Fragment in the Vector

In addition, part D ("Production of PB92 Variants") includes a description of the construction of various PB92 variants that were found to be useful in the present invention.

### A. Construction of Mutagenesis Vector M13M1

The basic construct pM58 was digested with restriction enzymes *Hpa*I and *Bal*I. The 1400 bp fragment containing the PB92 protease gene was purified on low melting agarose as known in the art. Vector M13MP11 *(See,* Messing et al., Nucl. Acids Res., 9:303-321 [1981]) was digested with *Sma*I. The 1400 bp DNA fragment of interest was ligated into this vector and transfected into *E. coli* JM101, using methods known in the art (*See,* Cohen et al., Proc. Natl. Acad. Sci. USA 69:2110-2114 [1972])

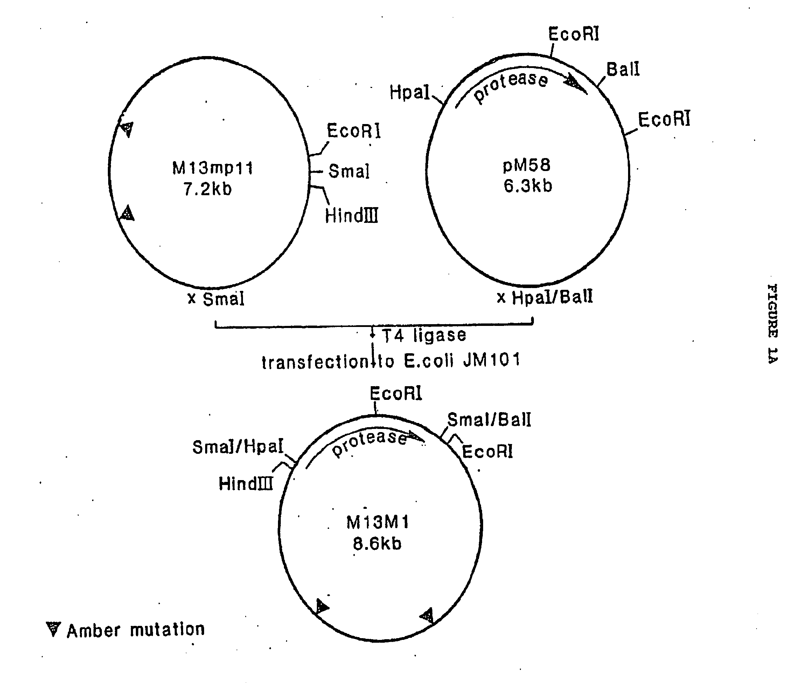

After phage propagation in *E. coli* JM101, ssDNA was isolated using methods known in the art (*See,* Heidecker et al., Gene 10:69-73 [1980], and the insert and its orientation were checked using Sanger DNA sequencing *(See,* Sanger, Proc. Natl. Acad. Sci. USA 74:6463 [1977]). The vector suitable for mutagenesis was obtained and named "M13M1." The procedure described above is schematically depicted in Figure 1A.

### B. Mutation Procedures

Mutagenesis was performed on M13M1 using ssDNA of this vector and dsDNA of M13mp19 (Messing et al. Nucl. Acids Res., 9:303-321 [1988]), which latter vector was digested with the restriction enzymes *Eco*RI and *Hind*III, followed by purification of the large fragment on low melting agarose.

Mutagenesis was performed as known in the art (*See,* Kramer et al., Nucl. Acids Res., 12:9441-9456 [1984]) with a modification being that *E. coli* JM105, rather than *E. coli* WK30-3 was used to select for mutants.

The length of the oligonucleotides used to create the specific mutations was 22 nucleotides. Region specific mutation used to create several mutations at the time in a specific DNA sequence, was performed using an oligonucleotide preparation with a length of 40 nucleotides with all four nucleotides randomly incorporated in the sites corresponding to the amino acid(s) to be mutated.

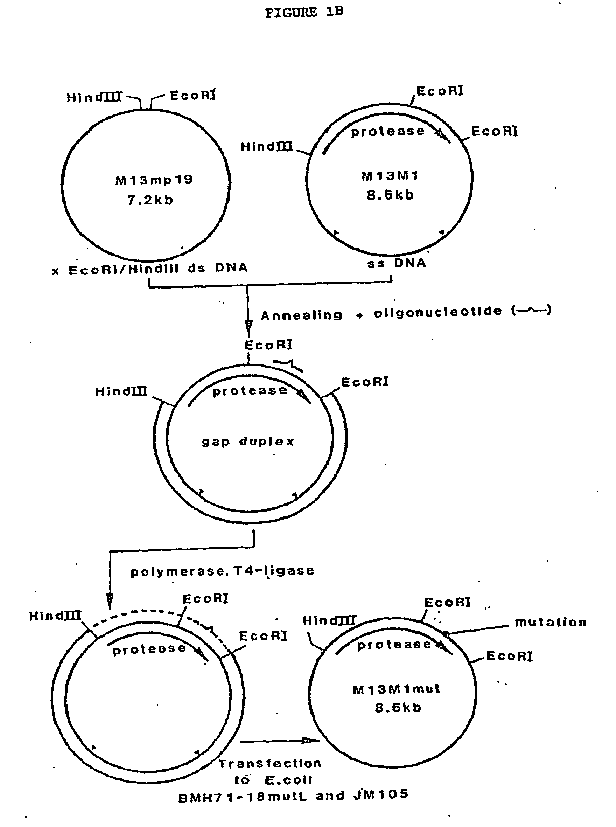

After mutagenesis, potential mutants were checked for the relevant mutation by sequence analysis using the Sanger dideoxy method *(See,* Sanger, *supra*). The entire single strand gap (*See,* Figure 1B) was sequenced to confirm the absence of secondary mutations. The procedure is schematically shown in Figure 1B.

The described procedure was useful for generating DNA fragments with mutations in the 3' part of the protease gene (amino acids 154-269).

However, it is not intended that the present invention be limited to these specific methods, as any suitable method known in the art will find use. Indeed, those skilled in the art recognize that in order to generate DNA fragments with mutations in the 5' part of the protease gene in a *Bacillus* vector, alternative restriction enzymes and modified PB92 protease genes find use in construction in methods that are analogous to the method illustrated in Figure 1A.

### C. Construction of pM58Eco and Subcloning of DNA Fragments Containing the Mutations in the Vector

To construct pM58Eco, pM58 was digested with restriction enzyme *Eco*RI and ligated with T4 ligase under diluted conditions, as known in the art. The ligation mixture was used to transform B. *subtilis* 1-A40 (Bacillus Genetic Stock Centre, Ohio) using methods known in the art *(See,* Spizizen et al., J. Bacteriol., 81:741-746 [1961]).

Cells from the transformation mixture were plated on minimal plates containing 20 g/ml neomycin as known in the art *(See,* Example 1 of EP 0283075).

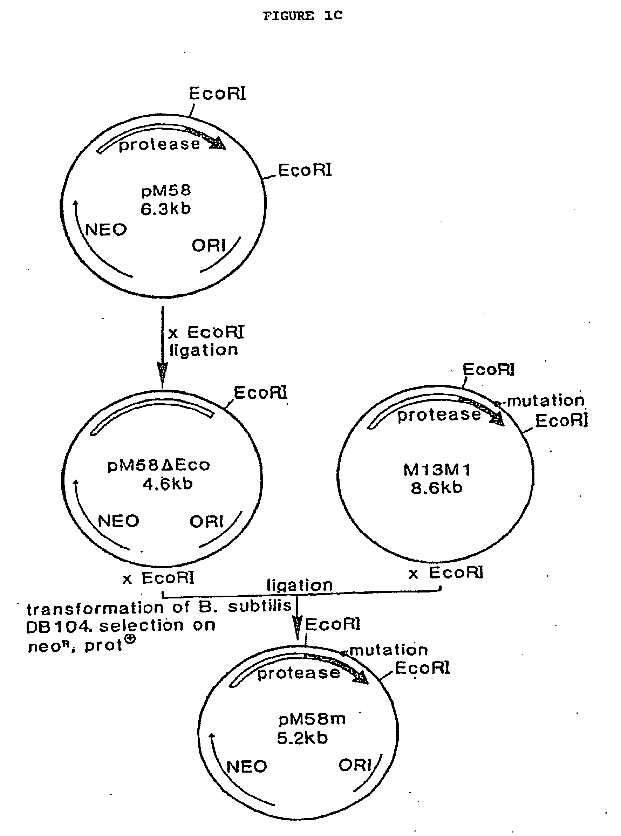

Plasmid DNA of transformants was isolated using methods known in the art (*See,* Birnboim and Doly, Nucl. Acids Res., 7:1513-1523 [1979]), and characterized using restriction enzyme analysis. Thus, in this manner, pM58Eco was isolated (*See,* Figure 1c).

To produce mutant enzyme, the DNA fragments of M13M1 containing the desired mutations generated as described in part B above, were subcloned into pM58Eco. Then, double-stranded DNA (dsDNA) of M13M1 (described above) was digested with *Eco*RI and ligated into the *Eco*RI site of pM58Eco. The ligation mixture was used to transform *B. subtilis* DB104 (*See,* Doi, J. Bacteriol., 160:442-444 [1984]) using methods known in the art (*See,* Spizizen *et al., supra*).

Cells from the transformation mixture were plated on minimal plates containing 20 g/ml neomycin and 0.4% casein as known in the art (*See,* EP 0283075). DNA of protease-producing transformants was isolated as known in the art (*See,* Birnboim and Doly, *supra*) and characterized by restriction enzyme analysis.

### D. Production of PB982 Variants

PB92 variants designated 053 through 060 were prepared by fusion PCR as described known in the art (*See e.g.,* US Pat. Appln. 2006/0252155 A1). The following Table provides the sequences of the primers used for fusion PCR as described herein.

| **Table 2. Primers Used in Fusion PCR** | |
|---|---|
| **Primer Sequence** | **Primer Name** |
| TCCTAAACTCAAATTAGCAACGTGCATGACATTGTTCCCT (SEQ ID NO:4) | 118V-Rv |
| ATGCACGTTGCTAATTTGAGTTTAGGA**TTCCTTACG**CCAAGTGCCACA (SEQ ID NO:5) | 128F-129L-130T-Fw |
| ATGCACGTTGCTAATTTGAGTTTAGGA**CTCAATGTG**CCAAGTGCCACA (SEQ ID NO:6) | 128L-129N-130V-Fw |
| ATGCACGTTGCTAATTTGAGTTTAGGA**TTCCAGTCG**CCAAGTGCCACA (SEQ ID NO:7) | 128F-129Q-Fw |
| ATGCACGTTGCTAATTTGAGTTTAGGACGCTCTCCGCCAAGTGCCACA (SEQ ID NO:8) | 128R-129S-130P-Fw |
| ATGCACGTTGCTAATTTGAGTTTAGGA**CGCCAGGAT**CCAAGTGCCACA (SEQ ID NO:9) | 128R-129Q-130D-Fw |
| ATGCACGTTGCTAATTTGAGTTTAGGA**TGCCGTGGG**CCAAGTGCCACA (SEQ ID NO:10) | 128C-129R-130G-Fw |
| TGCAGGCTCAATC**GAC**TATCCGGCCCGT (SEQ ID NO:11) | S166D-Fw |
| ACGGGCCGGATA**GTC**GATTGAGCCTGCA (SEQ ID NO:12) | S166D-Rv |
| GCAATTC**AGATCT**TCCTTCAGGTTATGACC (SEQ ID NO: 13) | pHPLT-BgIII-Fw |
| GCATCGA**AGATCT**GATTGCTTAACTGCTTC (SEQ ID NO:14) | pHPLT-BgIII-Rv |
| CCTAAACTCAAATTAGCAACGTGCATG (SEQ ID NO:15) | 128-up-Rv |

Phusion™ polymerase (Finnzymes) was used in these PCR reactions. In these experiments, 2 µl of 10mM forward and reverse primer, 1µl 10mM dNTP's, 5µl 10x HF Phusion buffer, 1.5µl DMSO and 1µl template was added to a volume of 50µl. The following program was used: 3 minutes denaturation at 95°C, annealing for 1 minute at 65°C, and elongation for 1 minute and 15 seconds at 72°C, for 30 cycles, followed by 7 minutes at 72°C. Following completion, the reaction products were stored at room temperature.

The mutant designated as "047/048" was used as template to develop mutants 053 through 058. The BgIII-Fw primer was combined with 118V-Rv, and the second fragment was prepared by combining the BgIII-Rv primer with the 128-130-Fw primers. In the case of 057, BgIII-Fw / S166D-Rv and BgIII-Rv / S166D-Fw were combined.

In addition, mutant "051/052" was used as template to create mutants 059 and 060. Primer 126-up-Rv was combined with BgIII-Fw, while BgIII-Rv was combined with 128-130Fw primers.

Fragments of the expected sizes were purified from agarose gels using PCR purification columns from Macherey-Nagel. The correct fragments were fused and amplified with the *Bgl*II primers using Phusion™ polymerase, and the following program: 3 minutes of denaturation time at 95°C, annealing for 1 minute at 65°C, and elongation for 2 minutes at 72°C for 25 cycles, followed by 7 minutes at 72°C. Following completion, the reaction products were stored at room temperature.

Fragments were digested with *Bgl*II, purified from agarose gels and ligated over night at 14°C with 1µl T4 DNA ligase, 8µl 5x T4 Ligation buffer in a volume of 40µl, using methods known in the art..

*B. subtilis* BG3594 comK highly transformable strain was used to obtain protease positive transformants. The expression vectors obtained as described above were transformed with 10 µl of the ligation product. Competent *Bacillus subtilis* cells, BG3594comK, were transformed with the expression plasmids, described in Example 1. The bacteria were made competent by the induction of the *comK* gene under control of axylose inducible promoter (*See e.g.,* Hahn et al., Mol. Microbiol., 21:763-775 [1996]). Protease positive clones were selected, isolated, sequenced and transformed to *B. clausii* PBT125 as described in Example 2.

### EXAMPLE 2

### Mutants Expressed in B. clausii

In this Example, methods used to develop mutant proteases expressed in *B. clausii* PBT125 transformed using the expression vectors described in Example 1 are provided. This strain is a protease negative derivative of PBT110, which was obtained from strain PB92 using classical strain improvement methods, followed by screening for asporogenity and improved protease production.

### Transformation Procedure of PB92 Protease-Negative Derivative: PBT125

The polyethylene glycol-induced protoplast transformation method of Chang and Cohen known in the art (*See e.g.,* Chang and Cohen, Mol. Gen. Genet., 168:111-115 [1979]), with the following modifications, was used to transform *B. clausii* PBT125 with the expression vectors described in Example 1. First, protoplasts were prepared in alkaline holding medium containing 0.5 M sucrose, 0.02 M MgCl₂, and 0.02 M Tris-maleate buffer (pH 8.0) to which 0.4 mg of lysozyme per ml was added. Then, the protoplasts were pelleted and suspended in 5 ml of alkaline holding medium to which 3.5% (wt/vol) Bacto-Penassay (Difco) broth and 0.04% albumin (Merieux) were added. The transformed protoplasts were regenerated on modified DM3 (5) plates containing 8.0 g of GELRITE® gellam Gum (Kelco), 0.3 g of CaCl₂ · 2H₂0, 4.06 g of MgCl₂ . H₂O, 5.0 g ofN-tris(hydroxymethyl)methyl-2-aminoethanesulfonic acid buffer (Sigma), 5.0 g of casamino acids, 5 g of yeast extract, 1.5 ml of 4 M NaOH dissolved in 750 ml of H₂O, and mixed after sterilization with 250 ml of 2 M sucrose and 10 ml of 50% (wt/vol) glucose, 1 ml of albumin (Merieux), 10 mg of thiamine, 5 mg of biotin, and 50 mg of neomycin. Plates were poured at approximately 70°C. Transformation of competent *B. clausii* cells was performed as known in the art (*See e.g.,* Tanaka, "Construction of Bacillus subtilis Plasmid and Molecular Cloning in Bacillus subtilis," In D. Schlesinger (ed.), Microbiology, American Society for Microbiology, Washington, D.C., pp. 15-18, [1982]).

### Fermentation Conditions and Protease Production

*B. clausii* PBT125 was fermented in 6 or 3-liter fermentors in a medium containing 22 g (based on dry matter) of yeast per liter, 5 g of K₂HPO₄ · 3H₂O per liter, 0.05 g of MgSO₄ · 7H₂O per liter, 0.05 g of CaCl₂ per liter, 0.005 g of FeSO₄ · 7H₂O per liter, and 0.05 g of MnSO₄ · 4H₂O per liter. The medium components were dissolved in 90% of the final volume and sterilized at pH 7.0 at a temperature of 120°C for 1 h. The inoculation culture was obtained by inoculation with *B. clausii* PBT125 into 100 ml of TSB; after sterilization, 4 ml of 1 M sodium carbonate solution was added from a slant tube. The inoculation culture was incubated at 37°C for 24 h on a shaking apparatus. The medium was inoculated at 37°C and a pH of 8.0 with 1 volume of the inoculation culture per 100 volumes of medium. The main fermentation was carried out at 37°C in stirred fermentors equipped with devices to control pH, temperature, and foaming and a device for continuous measurement of the dissolved oxygen concentration and the oxygen uptake rate. At 17 h after inoculation, a 30% glucose solution sterilized at 120°C for 1 h was added to a final concentration of 30 g of glucose per liter of medium.

The broths were spun for 30 minutes at 11,800 *xg.* The supernatant was filtered through a Whatman glasfibre filter and a 0.8 um filter in a Buchner funnel, followed by filtration using cellulosic pads. The resulting material was stored at 4°C, until used. Next, the material was concentrated using a Pall UF-filtration unit, with a filter cut-off of 10 kDa. The resulting UF-concentrate was formulated by adding propylene glycol and sodium formate. Formic acid was used to bring the pH to 6.0.

### EXAMPLE 3

### Analytical Techniques to Determine the Purity of Purified Proteases

In this Example, methods used to determine the purity of the purified proteases are described. Proteases were considered pure when a single band or peak was found by electrophoresis and high performance gel electrophoresis (HPLC), respectively.

Polyacrylamide gel-electrophoresis (PAGE) in the presence of sodium dodecyl sulphate (SDS) was conducted as known in the art (See, Laemmli, Nature, 227:680-685 [1970]). However, prior to denaturation of the protein samples by SDS at 100°C, inactivation of the protease activity was required, in order to prevent autodegradation. This was accomplished by incubation with phenylmethylsulfonyl fluoride (PMSF) (1 mM, 30 min, room temperature) or precipitation with trichloroacetic acid (TCA, 8%, 30 min, on ice). Native PAGE was carried out at pH 7.45 (gel buffer consisting of 20 mM histidine (His) and 50 mM 3-[N-morpholino]propanesulfonic acid (MOPS) in 5% polyacrylamide gels (ratio of acrylamide:bisacrylamide 20:1). Protein samples were loaded on top of slab gels and electrophoresed towards the cathode. The same 5 His/MOPS buffer was used as electrophoresis (tank) buffer, but at pH 6.3. After electrophoresis (1-2 h at 350 V), the gel was soaked in 8% acetic acid to fix the proteins in the gel and subsequently stained with Coomassie Brilliant Blue R250 and destained as known in the art.

The purity check by HPLC made use of a cation exchange column (MonoS; Pharmacia) and a gel filtration column (TSK 2000; SW-LKB). The former was run in a 10mM sodium phosphate buffer pH 5.5. Elution of the bound protease was obtained using a linear gradient of 10-300mM sodium phosphate, pH 5.5. The gel filtration column was run in 0.25M sodium acetate pH 5.5.

### EXAMPLE 4

### Determination of the Protease Concentration

In this Example, methods used to determine the protease concentrations are described. In some experiments extinction measurements were made at 280 nm using the calculated extinction coefficient (_{M}), and active site titration were used to determine the protein concentration in a purified protease solution., as described below. In additional experiments, the methods, set forth in U.S. Pat. 7,396,688

The extinction coefficient at 280 nm was calculated from the number of tryptophans (M = 5,600 M⁻¹ · cm⁻¹) and tyrosines (_{M} = 1,330 M⁻¹·cm⁻¹) per enzyme molecule. For PB92 protease, the _{M} was 26,100 M⁻¹ · cm⁻¹ (3 Trp, 7 Tyr residues) equivalent to ^{E1}1^{%} cm, measured at 280 nm = 9.7 (Mᵣ = 26,729 Da), was used. In the case of mutants with an altered number of Trp residues and Tyr residues, corrections were made accordingly.

An estimation of the number of active enzyme molecules was obtained with an active site titration. Since the widely used method with N-transcinnamoylimidazole (*See,* Bender et al., J. Am. Chem. Soc., 88:5890-5931 [1966]) proved not to work satisfactorily for PB92 protease, a method using PMSF was developed instead.

In this method, a protease solution with an estimated enzyme concentration (from the 280 nm absorption) was mixed with 0.25, 0.50, 0.75, 1.00 and 1.25 equivalents of PMSF, respectively, and allowed to react for one hour at room temperature in 10 mM sodium phosphate pH 6.5. The enzyme concentration had to be at least 50 M.

Residual activity was measured spectrophotometrically using succinyl-L-alanyl-L-alanyl-L-prolyl-L-phenylalanyl-para-nitroanilide (sAAPFpNA) as a substrate. The purity (and hence concentration) of PMSF was determined by NMR-spectroscopy and stock solutions were made in isopropanol. The results from the active site titration were found to be in agreement with the results from the purity check with HPLC.

### EXAMPLE 5

### Determination of Kinetic Parameters of Wild-Type and Mutant Proteases

In this Example, methods used to determine the kinetic parameters of wild-type and mutant proteases are described.

Activity on protein substrates (casein) was measured at pH 10.0 as described in British Patent Specification 1,353,317 (expressed in ADU's = Alkaline Delft Units).

The turnover number with casein as substrate was measured in a pH-stat. The reaction chamber of the pH-stat (Radiometer, Copenhagen) contained 10 ml 0.1 M KCl with 50 mg casein (Hammerstein, Merck). Protons, liberated upon hydrolysis of casein by PB92 protease were titrated with 10 mM NaOH, while the pH was maintained at 10.0 (at 40°C and under a flow of nitrogen gas).

Activity on synthetic peptides was measured using sAAPFpNA. The (yellow) paranitronanilide (pNA) formed was measured spectrophotometrically at 410 nm: M = 8,480 M⁻¹. cm⁻¹ using methods known in the art *(See,* Delmar et al., Anal. Biochem., 94:316-320 [1979]) with a UVIKON 860 (KONTRON) spectrophotometer equipped with a thermostat-controlled six position cell changer. The kinetic parameters kcat and Km were obtained from initial rate measurements at various substrate concentrations (for PB92 protease from 0.1-6.0 mM) and fitting the data to a hyperbolic function by non-linear regression using the multivariate secant iterative method. The specificity constant _{kcau/Km} was then calculated. Measurements were carried out at 25°C, in a final volume of 1 ml containing 0.1M TRIS-HCl + 0.1M NaCl pH 8.6. The sodium chloride was necessary, as in its absence, PB92 protease showed non-linear Lineweaver-Burk plots, that could have been caused by substrate inhibition. The substrate was first dissolved in DMSO to a concentration of 200mM and subsequently diluted with 0.1 M TRIS-HCl pH 8.6, to give a stock solution of 20mM (determined spectrophotometrically at 315 nm; M = 14,000 M⁻¹·cm⁻¹). No corrections were made for the varying concentrations of DMSO (0.05-3.0 % v/v).

### EXAMPLE 6

### Wash Performance Tests

In this Example, methods used to determine the wash performance of PB92 protease mutants and commercially available PROPERASE® serine protease in dishwashing applications using commercially available dish detergents are described.

In this Example, PB92 variants (049: G116V, S1261, P127Q, S128A, and 046: G116V, S1261, P127Q, S128A, S160D; PB92 numbering) were tested under various conditions. The compositions of the dish detergents are provided below. These detergents are commercially available from WFK and are referred to by the designations provided below. The protocols for each of the stain types (minced meat, egg yolk, and egg yolk with milk) are provided below. Before the individual soil types can be applied to the test dishes, the dishes must be thoroughly washed. This is particularly necessary, as residues of certain persistent stains may still be present on the dishes from previous tests. New dishes were also subjected to three thorough washes before being used for the first time in a test.

### Egg Yolk Stains on Stainless Steel

The stainless steel sheets (10x15 cm; brushed on one side) used in these experiments were thoroughly washed at 95°C in a laboratory dishwasher with a high-alkalinity commercial detergent (e.g., ECOLAB® detergent; Henkel) to provide sheets that were clean and grease-free. These sheets were deburred prior to their first use. The sheets were dried for 30 minutes at 80°C in a thermal cabinet before being soiled with egg yolk. The surfaces to be brushed were not touched prior to soiling. Also, no water stains or fluff on the surfaces were permitted. The cooled sheets were weighed before soiling.

The egg yolks were prepared by separating the yolks of approximately 10-11 eggs (200 g of egg yolk) from the whites. The yolks were stirred with a fork in a glass beaker to homogenize the yolk suspension. The yolks were then strained (approx. 0.5 mm mesh) to remove coarse particles and any egg shell fragments.

A flat brush (2.5") was used to apply 1.0 ± 0.1 g egg yolk suspension as uniformly as possible over an area of 140 cm² on the brushed sides of each of the stainless steel sheets, leaving an approx. 1 cm wide unsoiled rim (adhesive tape was used if needed). The soiled sheets were dried horizontally (to prevent formation of droplets on the edges of the sheets), at room temperature for 4 hours (max. 24 h).

For denaturation, the sheets were immersed for 30 seconds in boiling, demineralized water (using a holding device if necessary). Then, the sheets were dried again for 30 min at 80°C. After drying and cooling, the sheets were weighed. After weighing, the sheets were left for at least 24 hours (20°C, 40-60% relatively humidity) before submitting them to the wash test. In order to meet the testing requirements, only sheets with 500 ±100 mg/140 cm² (egg yolk after denaturation), were used in the testing. After the wash tests were conducted, the sheets were dried for 30 min at 80°C, in the thermal cabinet, and weighed again after cooling. The percent cleaning performance was determined by dividing the (mg of egg yolk released by washing x 100) by the (mg of denatured egg yolk applied).

### Minced Meat on Porcelain Plates

For these experiments, dessert plates (Arzberg, white, glazed porcelain) conforming to EN 50242, form 1495, No. 0219, diameter 19 cm were used. A total of 225 g lean pork and beef (half and half) was finely chopped and cooled, after removing visible fat. The mixture was twice run through a mincer. Temperatures above 35°C were avoided. Then, 225 g of the minced meat was mixed with 75 g of egg (white and yolk mixed together). The preparation was then frozen up to three months at -18°C, prior to use. If pork was not available, beef was used, as these are interchangeable.

The minced meat and egg mixture (300 g) was brought up to room temperature and mixed with 80 ml synthetic water. The mixture was then homogenized using a kitchen hand blender for 2 min. Then, a fork was used to spread 3 g of the minced meat/egg/water mixture on each white porcelain plate, leaving an approx. 2 cm wide unsoiled margin around the rim. The amount applied was 11.8 ± 0.5 mg/cm². The plates were dried for 2 hours at 120°C in a preheated thermal cabinet. As soon as the plates were cooled, they were ready for use. The plates were stacked with paper towels between each of the plates.

After washing, the plates were sprayed with ninhydrin solution (1% ethanol) for better identification of the minced meat residues. To promote the color reaction, the plates were heated for 10 min at 80°C in the thermal cabinet. Evaluation of the washing performance was done by visually inspecting the color reactions of the minced meat residues with reference to the IKW photographic catalogue (IKW).

### Egg/Milk Stains on Stainless Steel

The stainless steel sheets (10 x 15 cm; brushed on one side) used in these experiments were thoroughly washed at 95°C in a laboratory dishwasher with a high-alkalinity commercial detergent to remove grease and clean the sheets. The sheets were polished dry with a cellulose cloth. The surfaces to be brushed were not touched prior to soiling. Also, no water stains or fluff on the surfaces were permitted. Before soiling, the sheets were placed in a thermal cabinet at 80°C, for 30 min. The cooled sheets were weighed before soiling.

The egg yolks and whites of whole raw eggs (3-4 eggs; 160 g/egg) were placed in a bowl and beaten with an egg whisk. Then, 50 ml semi-skimmed UHT (1.5% fat, ultra-high temperature, homogenized) milk were added to the mixture. The milk and egg were mixed without generating froth. A flat brush was used to uniformly distribute 1.0 ± 0.1 g of the egg/milk mixture on the brushed side of the stainless steel sheets, using a balance to check the distribution. A margin of approximately 1.0 cm was left around the short sides of the sheets. The soiled sheets were dried horizontally (to prevent formation of droplets on the edges of the sheets), at room temperature for 4 hours (max. 24 h).

The sheets were then immersed for 30 seconds in boiling, demineralized water (using a holding device if necessary). Then, the sheets were dried again for 30 min at 80°C. After drying and cooling, the sheets were weighed. After weighing, the sheets were left for at least 24 hours (20°C, 40-60% relatively humidity) , before submitting them to the wash test. In order to meet the testing requirements, only sheets with 190 ± 10 mg egg yolk were used.

After the wash tests were conducted, the sheets were dried for 30 min at 80°C, in the thermal cabinet, and weighed again after cooling. The percentage cleaning performance was determined by dividing the (mg of egg/milk released by washing x 100) by the (mg of egg/milk applied).

### Washing Equipment and Conditions

The washing tests were performed in an automatic dishwasher (Miele: G690SC), equipped with soiled dishes and stainless steel sheets, as described above. A defined amount of the detergent was used, as indicated in the tables of results below. The temperatures tested were 45°C, 55°C and 65°C. The water hardness was 9° or 21° GH (German hardness) (374 ppm Ca).

As indicated above, after washing, the plates soiled with minced meat were visually assessed using a photo rating scale of from 0 to 10, wherein "0" designated a completely dirty plate and "10" designated a clean plate. These values correspond to the stain or soil removal (SR) capability of the enzyme-containing detergent.

The washed stainless steel plates soiled with egg yolk and/or egg yolk milk (were analyzed gravimetrically to determine the amount of residual stain after washing. The PB92 mutant protease and PROPERASE® protease and other mutants were tested at a level of between 0 and 20.57 mg/active protein per wash.

The detergents used in these experiments are described below. These detergents were obtained from the source without the presence of enzymes, to allow analysis of the enzymes tested in these experiments.

| **Phosphate-Free Detergent IEC-60436 WFK Type B (pH=10.4 in 3g/l)** | |
|---|---|
| **Component** | **Wt %** |
| Sodium citrate dehydrate | 30.0 |
| Maleic acid/ acrylic acid copolymer sodium Salt (SOKALAN® CP5; BASF) | 12.0 |
| Sodium perborate monohydrate | 5.0 |
| TAED | 2.0 |
| Sodium disilicate: Protil A (Cognis) | 25.0 |
| Linear fatty alcohol ethoxylate | 2.0 |
| Sodium carbonate anhydrous | add to 100 |

| **Phosphate-Containing Detergent: IEC-60436 WFK Type C (pH=10.5 in 3 g/l))** | |
|---|---|
| **Component** | **Wt %** |
| Sodium tripolyphosphate | 23.0 |
| Sodium citrate dehydrate | 22.3 |
| Maleic acid/ Acrylic Acis Copolymer Sodium Salt | 4.0 |
| Sodium perborate monohydrate | 6.0 |
| TAED | 2.0 |
| Sodium disilicate: Protil A (Cognis) | 5.0 |
| Linear Fatty Alcohol Ethoxylate | 2.0 |
| Sodium Carbonate anhydrous | add to 100 |

In the following Tables, the results for various experiments are provided. In each of these experiments, 20.57 mg active protein per wash were used. In these results, the index was 100. Thus, the performance results for PROPERASE® enzyme were assigned a value of "100," and the results for the mutants were compared to this value. For example, if PROPERASE® had a result of 45% SR (100 as index), and a mutant had a result of 52% SR, the result for the mutant would be 52/45 x 100 = 116 (as index).

| **Phosphate-Free Detergent, 45°C, 21°GH** | | | |
|---|---|---|---|
| **Enzyme** | **Wash performance on egg yolk, relative to PROPERASE®** | **Wash performance on minced meat, relative to PROPERASE®** | **Wash performance on egg yolk milk, relative to PROPERASE®** |
| PROPERASE® | 100 | 100 | 100 |
| PB92 046 | 71 | 104 | 88 |
| PB92 049 | 123 | 116 | 108 |

| **Phosphate-Free Detergent, 55°C, 21°GH** | | |
|---|---|---|
| **Enzyme** | **Wash Performance on Egg Yolk, Relative to PROPERASE®** | **Wash Performance on Minced Meat, Relative to PROPERASE®** |
| PROPERASE® | 100 | 100 |
| PB92 046 | ND | ND |
| PB92 049 | 134 | 128 |

| **Phosphate-Free Detergent, 45°C, 21°GH** | | | |
|---|---|---|---|
| **Enzyme** | **Wash Performance on Egg Yolk, Relative to PROPERASE®** | **Wash Performance on Minced Meat, Relative to PROPERASE®** | **Wash Performance on Egg Yolk Milk, Relative to PROPERASE®** |
| PROPERASE® | 100 | 100 | 100 |
| PB92 | 90 | 100 | 100 |
| PB92 046 | 104 | 71 | 88 |
| PB92 049 | 116 | 123 | 108 |

| **Phosphate-Containing Detergent, 55°C, 21°GH** | | | |
|---|---|---|---|
| **Enzyme** | **Wash Performance on Egg Yolk, Relative to PROPERASE®** | **Wash Performance on Minced Meat, Relative to PROPERASE®** | **Wash Performance on Egg Yolk Milk, Relative to PROPERASE®** |
| PROPERASE® | 100 | 100 | 100 |
| PB92 046 | 101 | 84 | 107 |
| PB92 049 | 125 | 114 | 111 |

| **Phosphate-Containing Detergent, 45°C, 21°GH** | | | |
|---|---|---|---|
| **Enzyme** | **Wash Performance on Egg Yolk, Relative to PROPERASE®** | **Wash Performance on Minced Meat, Relative to PROPERASE®** | **Wash Performance on Egg Yolk Milk, Relative to PROPERASE®** |
| PROPERASE® | 100 | 100 | 100 |
| PB92 046 | 94 | 76 | 104 |
| PB92 049 | 116 | 170 | 126 |

| **Phosphate-Containing Detergent, 65°C, 21°GH** | | | |
|---|---|---|---|
| **Enzyme** | **Wash Performance on Egg Yolk, Relative to PROPERASE®** | **Wash Performance on Minced Meat, Relative to PROPERASE®** | **Wash Performance on Egg Yolk Milk, Relative to PROPERASE®** |
| PROPERASE® | 100 | 100 | 100 |
| PB92 046 | 97 | 38 | 104 |
| PB92 049 | 122 | 126 | 112 |

## Claims

1. A dishwashing method, comprising the steps of
providing a dishwashing composition and dishware in need of cleaning; and contacting said dishware with said dishwashing composition under conditions effective to provide cleaning of said dishware;
wherein said dishwashing composition comprises a modified subtilisin, wherein said modified subtilisin has at least 70% homology with the sequence: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NGMHVANLSLGSPSPSATLEQAVNSATSRGVLVVAASGNSGAGSISYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR
 and comprises either
(i) the substitution G116V and at least one additional mutation; or
(ii) the substitution S126R.

2. The dishwashing method of Claim 1, wherein said modified subtilisin comprises substitutions made at positions G116, S126, P127, and S128.

3. The dishwashing method of Claim 1, wherein said subtilisin comprises the substitution G116V and the additional mutation is selected from the group consisting of S126F, S126L, S126N, S126R, S126V, P127E, P127L, P127M, P127N, P127L, P127Q, P127S, S128A, S128K, S128P, S128T, S128V, and S160D.

4. The dishwashing method of Claim 1, wherein said modified subtilisin comprises substitutions made at positions S126, P127, and S128.

5. The dishwashing method of Claim 4, wherein said substitutions are selected from the group consisting of S126C, S126R, P127Q, P127R, S128D, and S128G.

6. The dishwashing method of Claim 1, wherein the amino acid sequence of said modified subtilisin is: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSISYPARYANAMAV
 
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR
 or

AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSIDYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR.

## Patentansprüche

1. Geschirrspülverfahren, das die folgenden Schritte umfasst: das Bereitstellen einer Geschirrspülzusammensetzung und von Geschirr mit Reinigungsbedarf und das Kontaktieren des Geschirrs mit der Geschirrspülzusammensetzung unter zur Bereitstellung von Reinigung des Geschirrs wirksamen Bedingungen,
worin die Geschirrspülzusammensetzung ein modifiziertes Subtilisin umfasst, worin das modifizierte Subtilisin zumindest 70 % Homologie mit der Sequenz 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NGMHVANLSLGSPSPSATLEQAVNSATSRGVLVVAASGNSGAGSISYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR
 aufweist und entweder
(i) die Substitution G116V und zumindest eine weitere Mutation oder
(ii) die Substitution S126R
umfasst.

2. Geschirrspülverfahren nach Anspruch 1, worin das modifizierte Subtilisin an den Positionen G116, S126, P127 und S128 vorgenommene Substitutionen umfasst.

3. Geschirrspülverfahren nach Anspruch 1, worin das Subtilisin die Substitution G116V umfasst und die weitere Mutation aus der aus S126F, S126L, S126N, S126R, S126V, P127E, P127L, P127M, P127N, P127L, P127Q, P127S, S128A, S128K, S128P, S128T, S128V und S160D bestehenden Gruppe ausgewählt ist.

4. Geschirrspülverfahren nach Anspruch 1, worin das modifizierte Subtilisin an den Positionen S126, P127 und S128 vorgenommene Substitutionen umfasst.

5. Geschirrspülverfahren nach Anspruch 4, worin die Substitutionen aus der aus S126C, S126R, P127Q, P127R, S128D und S128G bestehenden Gruppe ausgewählt sind.

6. Geschirrspülverfahren nach Anspruch 1, worin die Aminosäuresequenz des modifizierten Subtilisins wie folgt lautet: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSISYPARYANAMAV

GATDQNNNRASFSQYGAGLDIVAPGVNVQSTYPGSTYASLNGTSMATPHVAGAAALV
KOKNPSWSNVOIRNHLKNTATSLGSTNLYGSGLVNAEAATR
 oder

AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NVMHVANLSLGLQAPSATLEQAVNSATSRGVLVVAASGNSGAGSIDYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR.

## Revendications

1. Procédé de lavage de vaisselle, comprenant les étapes de:
réaliser une composition pour lavage de vaisselle et la vaisselle devant être nettoyée; et
mettre en contact ladite vaisselle avec ladite composition pour lavage de vaisselle sous des conditions efficaces pour provoquer le nettoyage de ladite vaisselle;
où ladite composition pour lavage de vaisselle comprend une subtilisine modifiée, où ladite subtilisine modifiée a au moins 70% d'homologie avec la séquence: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NGMHVANLSLGSPSPSATLEQAVNSATSRGVLVVAASGNSGAGSISYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR
 et comprend soit
(i) la substitution G116V et au moins une mutation additionnelle; ou
(ii) la substitution S126R.

2. Procédé de lavage de vaisselle selon la revendication 1, où ladite subtilisine modifiée comprend des substitutions faites aux positions G116, S126, P127 et S128.

3. Procédé de lavage de vaisselle selon la revendication 1, où ladite subtilisine comprend la substitution de G116V, et la mutation additionnelle est sélectionnée dans le groupe consistant en S126F, S126L, S126N, S126R, S126V, P127E, P127L, P127M, P127N, P127L, P127Q, P127S, S128A, S128K, S128P, S128T, S128V et S160D.

4. Procédé de lavage de vaisselle selon la revendication 1, où ladite subtilisine modifiée comprend des substitutions faites aux positions S126, P127 et S128.

5. Procédé de lavage de vaisselle selon la revendication 4, où lesdites substitutions sont sélectionnées dans le groupe consistant en S126C, S126R, P127Q, P127R, S128D et S128G.

6. Procédé de lavage de vaisselle selon la revendication 1, où la séquence d'acides aminés de ladite subtilisine modifiée est: 
AQSVPWGISRVQAPAAHNRGLTGSGVKVAVLDTGISTHPDLNIRGGASFVPGEPSTQ
DGNGHGTHVAGTIAALNNSIGVLGVAPNAELYAVKVLGASGSGSVSSIAQGLEWAGN
NGMHVANLSLGSPSPSATLEQAVNSATSRGVLVVAASGNSGAGSISYPARYANAMAV
GATDQNNNRASFSQYGAGLDIVAPGVNVQSTYPGSTYASLNGTSMATPHVAGAAALV
KQKNPSWSNVQIRNHLKNTATSLGSTNLYGSGLVNAEAATR
 ou
